# EUROPEAN PATENT SPECIFICATION

(11) **EP 3 862 428 B1**
(45) Date of publication and mention of the grant of the patent: **28.02.2024**
(21) Application number: 19868942.4
(22) Date of filing: 10.09.2019
(51) Int. Cl.: C12N 13/00, C12N 5/077

(54) **METHOD FOR EXTENDING TELOMERE OF CELL**
VERFAHREN ZUR VERLÄNGERUNG DES TELOMERS EINER ZELLE
PROCÉDÉ D'ALLONGEMENT DE TELOMÈRE CELLULAIRE

(30) Priority: 02.10.2018 KR 20180117265; 03.09.2019 KR 20190108764
(43) Date of publication of application: 11.08.2021
(73) Proprietor: STEMON Inc., Gyeonggi-do 13486 (KR)
(72) Inventor: LEE, Yong Seung, Suwon-si Gyeonggi-do 16591 (KR)
(74) Representative: M. Zardi & Co S.A.
(86) International application number: PCT/KR2019/011681
(87) International publication number: WO 2020/071652

(56) References cited:
- WO-A1-95/13383
- WO-A1-2016/000075
- WO-A1-2018/057542
- WO-A2-2006/066247
- KR-B1- 101 855 967
- US-A1- 2014 242 155
- BROCK J. SISHC ET AL: "Telomeres and Telomerase in the Radiation Response: Implications for Instability, Reprograming, and Carcinogenesis", FRONTIERS IN ONCOLOGY, vol. 5, 24 November 2015 (2015-11-24), pages 1-19, XP055586183, DOI: 10.3389/fonc.2015.00257
- NEUHOF D ET AL: "Telomerase activity and telomere length in human lymphoblasts after irradiation", INTERNATIONAL JOURNAL OF RADIATION: ONCOLOGY BIOLOGY PHYSICS, PERGAMON PRESS, USA, vol. 48, no. 3, 1 January 2000 (2000-01-01), page 276, XP027402761, ISSN: 0360-3016 [retrieved on 2000-01-01]
- V KH KHAVINSON ET AL: "Epithalon Peptide Induces Telomerase Activity and Telomere Elongation in Human Somatic Cells", BULLETIN OF EXPERIMENTAL BIOLOGY AND MEDICINE, vol. 135, no. 6, 1 June 2003 (2003-06-01), pages 590-592, XP055579199, US ISSN: 0007-4888, DOI: 10.1023/A:1025493705728
- MURILLO-ORTIZ, B. et al.: "Increased telomere length and proliferative potential in peripheral blood mononuclear cells of adults of different ages stimulated with concanavalin A", BMC Geriatrics, vol. 13, 2013, pages 1-5, XP021163371,
- KHAVINSON, V. K. et al.: "Epithalon peptide induces telomerase activity and telomere elongation in human somatic cells", Bulletin of Experimental Biology and Medicine, vol. 135, no. 6, June 2003 (2003-06), pages 590-592, XP055579199, DOI: 10.1023/A:1025493705728

## Description

### [Technical Field]

The present invention relates to a method for elongating telomeres of cells, and more particularly, to a method of elongating telomeres of cells by providing physical stimulation directly or indirectly to the cells.

### [Background Art]

Telomeres are DNA repeat structures (TTAGGG in humans) at both ends of chromosomes. Telomeres bind to the shelterin complex to form a protective cap, which regulates the multiplication ability of cells and prevents binding between chromosomes and loss of genetic information during cell division. Since DNA polymerase cannot completely amplify the 3' end, telomeres are shortened by 30 to 200 bp for each cell division. When telomeres become shorter than the threshold value and become closer to the coding DNA and the loop structure of the telomeres cannot be maintained, the exposed telomeres are recognized by the p53 or p16INK4a signaling pathways, so that cell division stops and the cells die due to senescence.

Telomeres may be elongated by the reverse transcriptase telomerase, and the human telomerase complex is composed of TERC, which is an RNA molecule that acts as a template for telomere synthesis, TERT, which is a catalytic subunit, and telomerase-associated proteins such as DKC1 and TEP1. In normal cases, somatic cells have little telomerase activity, and high telomerase activity is detected only in stem cells and progenitor cells that require active division ability.

When the division of multiple cells is stopped as the telomeres are shortened, various problems may arise, and in particular, various symptoms represented by aging and degeneration may arise. It was already known that diseases that cause problems in the regeneration of bone marrow cells, such as congenital dyskeratosis congenita in which skin tissue is degenerated, and aplastic anemia in which blood cells counts are low, arise due to abnormalities in telomeres maintenance, such as mutations in telomerase or Shelterin complex-related genes containing the same. In recent years, studies have been reported that telomeres in patients suffering from aging-related diseases such as hypertension, metabolic syndrome, diabetes, and dementia are shorter than those in normal people, and thus studies on the possibility of slowing aging by increasing the length of telomeres have attracted attention. In addition, it has been shown that regenerative activities can be actively performed by increasing telomerase activity in somatic cells, which are generally known to have little telomerase activity. In particular, it has been reported that the increase in telomerase activity is effective when it is necessary to supplement various tissues such as the myocardium, liver, cornea, skin, blood vessels, cartilage and bone, immune cells, blood cells, etc. due to bums, injuries, aging and diseases. However, studies have also been reported that a number of cancer cells occurred in mice in which a gene capable of extending the length of telomeres was continuously overexpressed, suggesting that long telomeres become a risk factor for cancer. Therefore, it can be expected that, if telomeres can be temporarily elongated, this telomere elongation can alleviate various symptoms associated with aging and degeneration without the risk of cancer development, and can be particularly useful in the field of regenerative medicine.

According to the various utilities expected as described above, various methods for elongating telomeres have been developed. US Patent Application Publication No. 2018-0280413 discloses a pharmaceutical composition containing a compound for enhancing telomerase activity. However, in this case, a process of synthesizing the compound is required, and the time for the composition to exhibit the effect thereof is long. In addition, the effect of this compound has been shown to last for a considerable amount of time, but it is known that there is a risk of cancer development if the telomerase activity is continuously high. European Patent Publication No. 2959005 discloses a nucleic acid encoding telomerase. In this case, a synthesis process is also required, and since the nucleic acid is hydrophilic, a delivery vehicle is additionally required to deliver the nucleic acid into cells. Korean Patent Application Publication No. 10-2018-0123512 discloses a telomerase-derived peptide. In this case, a synthesis process is also required, and this peptide exhibits an effect when measured 1 month after injection 3 times a week for 2 months, and thus can be cumbersome and costly.

The publication WO 2016/000075 A1 describes a device for generating ultrasound vibrations for extending the length of telomeres; this document is silent on application to cells and concentrates on entire organisms. The publication WO 2006/066247 A2 describes a method for elongating cell telomeres by enhancing hTERT expression via the creation of hypoxic conditions. The publication Frontiers in Oncology, vol. 5, 2015, pp.1-19 describes the treatment of PBMCs with LDR gamma radiation, obtaining a high telomerase activity associated to both shortening and lengthening of telomeres. The publication International Journal of Radiation: Oncology, Biology Physics, vol. 48(3), 2000, page 276 describes an increased telomerase activity and telomere length after irradiation in two closely related human lymphoblast cell lines. The publication WO2018/057542 relates to extension of telomere length using a composition comprising cardiosphere-derived cells and extracellular vesicles. The publication Bullettin of Experimental Biology and Medicine, vol. 135(6), 2003, pp.590-592 relates to telomere elongation in human somatic cells using the epithalon peptide.

Therefore, there is a need for a method for elongating telomeres, which is more reasonable in terms of cost and time of synthesis, etc., and is safe and more efficient.

### Technical Problem]

The present invention has been made in order to solve the above-described problems, and provides a method for elongating telomeres.

### [Technical Solution]

As a technical means for achieving the above-described technical problem, the method of the invention comprises the steps of: providing physical stimulation directly or indirectly to the cells; and culturing a mixture of the cells and a medium for a predetermined time. Providing the stimulation directly to the cells is performed by applying the physical stimulation to a medium containing the cells; providing the stimulation indirectly to the cells is performed by applying the physical stimulation to the medium not containing a cells and then mixing the medium and the cells. The physical stimulation is direct or indirect ultrasound stimulation; the direct stimulation is performed at an ultrasound intensity of 0.1 to 3 W/cm2 and a frequency of 20 kHz to 20 MHz for a duration of 0.1 seconds to 20 minutes; the indirect stimulation is performed at an ultrasound intensity of 1 to 20 W/cm2 and a frequency of 20 kHz to 20 MHz for a duration of 0.1 seconds to 20 minutes.

The step of providing physical stimulation directly or indirectly to cells may be performed by any one method selected from among: a method of mixing the cells and a medium and then providing physical stimulation to the mixture; or a method of providing physical stimulation to a medium and then mixing the medium and the cells, optionally followed by providing physical stimulation to the mixture.

The physical stimulation is ultrasound stimulation, and providing the ultrasound stimulation directly to the cells is performed at an ultrasound intensity of 0.1 to 3 W/cm² and a frequency of 20 kHz to 20 MHz for a duration of 0.1 seconds to 20 minutes, and providing the ultrasound stimulation indirectly to the cells may be performed at an ultrasound intensity of 1 to 20 W/cm² and a frequency of 20 kHz to 20 MHz for a duration of 0.1 seconds to 20 minutes.

The cells may be selected from the group consisting of mammalian stem cells, progenitor cells, fibroblasts, keratinocytes or organ tissue cells.

The medium may be selected from a culture medium or a differentiation-inducing medium.

The culturing of the mixture may be performed for 1 hour to 10 days.

Expression of one or more of TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin in the cells after the culturing may increase compared to that before the culturing.

Telomerase activity in the cells after the culturing may increase compared to that before the culturing.

β-galactosidase activity in the cells after the culturing may decrease compared to that before the culturing.

The present method provides cells having telomeres elongated by physical stimulation.

### [Advantageous Effects]

The method for elongating telomeres of cells according to one embodiment of the present invention is simpler and more cost-effective than the above-described conventional methods of elongating telomeres. That is, the conventional methods are methods of administering compounds, and in these methods, a process of synthesizing and purifying these compounds using chemical or biological methods is required, and equipment therefor and materials that must be continuously fed are also required. Chemical synthesis has no specificity and is harmful to the environment, and these shortcomings can be overcome by biosynthesis, but biosynthesis has disadvantages in that the yield is low, mass production and quality maintenance are difficult, the possibility of contamination is high, and thus additional equipment and materials are required to overcome this contamination. In addition, since cells are surrounded by a double lipid membrane, a delivery vehicle capable of delivering these compounds into cells is additionally required depending on the chemical properties of the purified compounds, and in this case, a step of loading the compound into the delivery vehicle is required. In contrast, the method for elongating telomeres of cells according to one embodiment of the present invention exhibits an effect by directly treating the cells with physical stimulation, and thus makes it possible to omit processes such as synthesis, purification, delivery vehicle design and delivery vehicle synthesis. In addition, since the method of the present invention may be carried out as long as there is equipment capable of applying stimulation, it can elongate telomeres at low cost.

In addition, the method for elongating telomeres of cells according to the present invention is superior in terms of time and efficiency compared to the previously disclosed method of elongating telomeres. In the process of synthesizing the telomereelongating compound according to the conventional technology, raw materials are fed, but not all of them constitute a product, and considerable amounts of materials such as by-products or buffers are discarded. However, in the method of the present invention, there is no such waste, because the method has no process of synthesizing a compound and is a method of applying physical stimulation. In addition, the above-described methods of elongating telomeres the compounds require treating cells with a high concentration of a compound several times or over a long period of time in order to exhibit a sufficient effect, whereas the method for elongating telomeres of cells according to the present invention exhibits an effect by treating cells once with physical stimulation for a short duration of 20 minutes or less.

In addition, the method of elongating telomeres of cells according to the present invention has high safety. That is, the compound may pose additional risks such as toxicity in the metabolic process, and in the above-described conventional technology, the dosage of the compound is considerably high (in the order of mg/kg), waste that burden the environment is generated in the synthesis process, whereas the method according to the present invention does not involve these problems. In addition, the method according to the present invention temporarily induces increased expression of the TERT gene related to telomere elongation by performing ultrasound treatment for a short time. Since continuous expression of the gene is known to be correlated with cancer development, it is expected that, when cells whose telomeres have been elongated by the method of the present invention, in which the effect of increasing the expression of the gene is not long-lasting, are administered for therapeutic purposes or applied to a living body, they will be safer from the risk of cancer development

In addition, it was confirmed that the method of elongating telomeres of cells according to the present invention induces cell division and provides an anti-aging effect, in addition to simply elongating telomeres. Thereby, it is expected that the method of the present invention can ameliorate and prevent not only problems caused by shortening of telomeres, but also various aging-related diseases and conditions.

### [Description of Drawings]

FIG. 1 shows data obtained by analyzing telomere length changes depending on the type of cells after ultrasound treatment and culture according to Example 1 of the present invention.
FIG. 2 shows data obtained by analyzing telomere length changes caused by each physical stimulation treatment after ultrasound stimulation treatment and culture according to Example 1 of the present invention, heat stimulation treatment and culture according to Example 2, and light-emitting diode laser light stimulation treatment and culture according to Example 3.
FIG. 3 shows data obtained by analyzing telomere length changes after indirect ultrasound stimulation treatment and culture according to Example 5.
FIG. 4 shows data obtained by analyzing telomere length changes depending on the kind of medium after ultrasound treatment and culture according to Example 1 of the present invention.
FIG. 5 shows data obtained by analyzing telomere length changes depending on the number of ultrasound treatments after ultrasound treatment and culture according to Example 1 of the present invention.
FIG. 6 shows data obtained by analyzing changes in TERT gene expression depending on the type of cells after ultrasound treatment and culture of the cells according to Example 1 of the present invention.
FIG. 7 shows data obtained by analyzing changes in TERT gene expression in CB-HDFs and Adipo-MSC cells after ultrasound treatment and culture of the CB-HDFs and Adipo-MSC cells according to Example 1 of the present invention.
FIG. 8 shows data obtained by analyzing changes in β-catenin gene expression in CB-HDFs and Adipo-MSC cells after ultrasound treatment and culture of the CB-HDFs and Adipo-MSC cells according to Example 1 of the present invention.
FIG. 9 shows data obtained by analyzing telomerase activity changes in CB-HDFs and Adipo-MSC cells after ultrasound treatment and culture of the CB-HDFs and Adipo-MSC cells according to Example 1 of the present invention.
FIG. 10 shows data obtained by performing FISH analysis of telomeres in CB-HDFs and Adipo-MSC cells after ultrasound treatment and culture according to Example 1 of the present invention.
FIG. 11 shows data obtained by performing fluorescence staining for TERT and Ki67 in CB-HDFs after ultrasound treatment and culture of the CB-HDFs according to Example 1 of the present invention.
FIG. 12 shows data obtained by analyzing senescence-related β-galactosidase activity in CB-HDFs after ultrasound treatment and culture of the CB-HDFs according to Example 1 of the present invention.

### [Mode for Invention]

Hereinafter, the present invention will be described in more detail.

The terminology used herein is only for the purpose of describing particular embodiments and is not intended to be limiting of the present invention. Singular expressions include plural expressions unless otherwise specified in the context thereof. Throughout the present specification, it is to be understood that when any part is referred to as "comprising" any component, it does not exclude other components, but may further comprise other components, unless otherwise specified.

In addition, the unique name of each gene used under the designation "gene" in the present invention is an officially known gene name, a commonly used name, or the name of a product of the gene, for example, a protein in the case of the gene encoding the protein.

The present inventors previously disclosed in Korean Patent No. 10-1855967 that, when physical stimulation capable of promoting environmental inflow is provided to a mixture of cells and a culture medium and the mixture provided with the physical stimulation is cultured for a predetermined time, reprogrammed cells can be obtained. Here, the direction of reprogramming appears differently depending on the composition of the medium regardless of the type of cell. In the process of analyzing the effect of the cell reprogramming method, it was confirmed that the expression of the gene group related to telomere elongation was also increased by applying physical stimulation. Through further research thereon, it has been found that, unlike the previously disclosed invention described above, telomeres can be elongated by physical stimulation regardless of not only the type of cells tested but also the composition of medium. Based on this finding, it was possible to conclude that telomere elongation was induced by the physical stimulation itself rather than the environmental inflow. Based on this conclusion, a new invention is disclosed.

The physical stimulation may be provided directly or indirectly to the cells one or more times, and as the number of times increases, the telomere elongation effect may increase proportionally. When the physical stimulation is provided one or more times as described above, it is preferable to provide a time interval between the provisions so that the cells can recover, and the time interval may be at least 1 day, more preferably at least 2 days.

The physical stimulation is ultrasound stimulation. Providing the ultrasound stimulation directly to the cells is performed at an ultrasound intensity of 0.1 to 3 W/cm² and a frequency of 20 kHz to 20 MHz for a duration of 0.1 seconds to 20 minutes, and providing the ultrasound stimulation indirectly to the cells is performed at an ultrasound intensity of 1 to 20 W/cm² and a frequency of 20 kHz to 20 MHz for a duration of 0.1 seconds to 20 minutes. Preferably, providing the ultrasound stimulation directly to the cells may be performed at an ultrasound intensity of 0.5 to 2 W/cm² and a frequency of 20 kHz to 2 MHz for a duration of 0.1 seconds to 10 minutes, and providing the ultrasound stimulation indirectly to the cells may be performed at an ultrasound intensity of 2 to 10 W/cm² and a frequency of 20 kHz to 2 MHz for a duration of 1 second to 15 minutes.

It was confirmed that, when the method for elongating telomeres of cells according to one embodiment of the present disclosure was applied to various types of cells, it could elongate telomeres of each type of cells. The cells may be selected from the group consisting of mammalian stem cells, progenitor cells, fibroblasts, keratinocytes, or organ tissue cells. When the cells having elongated telomeres are used for *in vivo* application, the cells may be either autologous, allogeneic or heterologous. When the cells are heterologous, the cells may be of mammalian origin. In order to reduce the likelihood of immune rejection, the cells are preferably allogeneic cells, most preferably autologous cells.

The medium may be selected from a culture medium or a differentiation-inducing medium. Here, the term "culture medium" refers to a medium optimized for survival of a specific type of cells while maintaining the uniformity of the cells, which is a medium that is used for uniform cell proliferation. On the other hand, the term "differentiation inducing medium" refers to a medium for inducing differentiation of a specific type of cells into another type of cells having other differentiation potentials or functions.

The culturing of the mixture may be performed for 1 hour to 10 days, preferably 1 to 5 days. The reason therefor is as follows. When the above-described physical stimulation is applied to cells, the expression of various genes described later is increased, and in this case, "gene expression" involves transcription, the synthesis and folding of a bioactive substance such as a protein, and the migration of the substance to a necessary position in the cell, and hence it takes time to actually elongate telomeres.

Expression of one or more of TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin genes in the cells after the culturing may increase compared to that before the culturing. TERT (telomerase reverse transcriptase) is a subunit having telomerase catalytic activity, and TERF1 (telomeric repeat binding factor 1) and TERF2 (telomeric repeat binding factor 2) recognize the telomere sequence. It is known that DKC1 (dyskerin pseudouridine synthase 1), RFC1 (replication factor C subunit 1), TNKS1BP1 (tankyrase 1 binding protein 1), NBN (Nibrin), HSPA1L (heat shock protein family A member 1 like), PARP1 (poly ADP-ribose polymerase 1), PTGES3 (prostaglandin E synthase 3), SMG6 (Smg6 homolog, Nonsense mediated mRNA decay factor), XRCC5 (X-ray repair cross complementing 5) and XRCC6 (X-ray repair cross complementing 6) are necessary for stabilization and maintenance of telomeres. It has been reported that TERF2IP (TERF2 interacting protein) and RAD50 (RAD50 homolog, double strand break repair protein) inhibit telomere recombination, and PINX1 (PIN2/TERF1-interacting telomerase inhibitor 1) mediate the accumulation of TERF1 and TERT in the nucleolus, help TRF1 bind to telomerase, and inhibit telomerase activity in the S phase. ACD (adrenocortical dysplasia protein homolog), ERCC4 (excision repair cross-complementation group 4) and PRKDC (protein kinase, DNA-activated, catalytic subunit) are necessary to control the telomere length and for telomere protection, and promote telomere amplification during BLM (bloom syndrome protein) DNA synthesis, and telomerase associated protein 1 (TEP1) forms part of the telomerase complex. β-Catenin is a transcriptional regulator that promotes TERT expression. The method for elongating telomeres according to the present invention may be used for various purposes as described below.

Telomerase activity in the cells after the culturing may increase compared to that before the culturing. This increase in telomerase activity may be at least 1.5 times higher after 2 days of the culturing than before the culturing, and as the telomerase activity increases in this manner, the telomere length may increase 1.2 times or more.

β-galactosidase activity in the cells after the culturing may decrease compared to that before the culturing. β-galactosidase is an enzyme that catalyzes the hydrolysis of β-galactoside into monosaccharides, and the lysosomal β-galactosidase is overexpressed and accumulated in senescent cells. As a result, it has been reported that the activity of β-galactosidase in senescent cells is high. It was confirmed that the activity of the enzyme was lowered by the method for elongating telomeres of cells according to one embodiment of the present invention, suggesting that the method has an anti-aging effect.

The present disclosure provides cells having telomeres elongated by physical stimulation. The present inventors have found that, when cells are treated with physical stimulation, telomeres of the cells are elongated while the expression of telomerase activity-related genes, such as TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin, in the cells, is increased, and exosomes containing the mRNAs and proteins of these genes are secreted in large amounts. Exosomes containing various factors such as mRNAs and proteins may migrate along the bloodstream and exhibit therapeutic effects by delivering the factors directly to cells. Thus, the method for elongating telomeres of cells according to the present invention, the cells and exosomes derived from these cells may be used for therapeutic purposes in a subject in need of the activities of these genes. For example, they may be used for the treatment or amelioration of diseases known to occur due to abnormal telomere maintenance, such as congenital dysphagia, aplastic anemia, Werner syndrome, Bloom syndrome, Ataxia-telangiectasia, Nijmegen breakage syndrome, Ataxia-telangiectasia-like syndrome, and pulmonary fibrosis. In addition, they may be used for the treatment or amelioration of various diseases and conditions caused by cell senescence and loss, for example, various degenerative diseases such as dementia, ataxia, and degeneration of cartilage and bone, autoimmune diseases such as Crohn's disease and chronic obstructive pulmonary disease, hypertension, metabolic syndrome, diabetes, skin aging, pigmentation abnormalities, hair loss, etc. In addition, they may be used for rapid regeneration from various injuries such as bums, wounds, injuries, organ failure, organ loss, and ulcers.

Hereinafter, examples of the present invention will be described in detail so that the present invention can be easily carried out by those skilled in the art. However, the present invention may be embodied in various different forms.

All cell culture processes in the Examples and Experimental Examples of the present invention were performed at 37°C under 5% CO₂.

### Example 1. Induction of Telomere Elongation in Cells by Direct Ultrasound Stimulation

Ultrasound stimulation was applied to 1×10⁶ cells in 1 ml of medium at an ultrasound intensity of 1 W/cm² and a frequency of 20 KHz for 5 seconds, and the cells were cultured with the same medium in a culture dish for 1 day or more.

### Example 2. Induction of Telomere Elongation in Cells by Direct Heat Stimulation (not part of the invention)

1 × 10⁶cells in 1 ml of hES medium were placed in a 1.5-ml tube and exposed to 50°C for 5 seconds and then exposed to 0°C for 10 seconds, and the cells were cultured with the same medium in a culture dish for 1 day or more.

### Example 3. Induction of Telomere Elongation in Cells by Direct Light-Emitting Diode Light Stimulation (not part of the invention)

Light-emitting diode light stimulation (1 W, 500 Hz/sec) with a wavelength of 808 nm was applied directly to 1×10⁶ cells in 1 ml of hES medium for 5 seconds, and the cells were cultured with the same medium in a culture dish for 1 day or more.

### Example 4. Induction of Telomere Elongation in Cells by Indirect Ultrasound Stimulation

hES medium was treated with ultrasound at 0, 3, 5 and 10 W/cm² for 10 minutes and then mixed with non-ultrasound-treated cells, followed by 1 day or more of culture.

### Example 5. Production of Exosomes Capable of inducing Cell Telomere Elongation by Ultrasound Stimulation

Ultrasound stimulation was applied to 1×10⁶ cells in 1 ml of medium at an ultrasound intensity of 1 W/cm² and a frequency of 20 KHz for 5 seconds, and the cells were cultured with the same medium in a culture dish for 1 to 2 days. Next, the conditioned medium was collected and centrifuged at 3000 rpm for 5 minutes to remove cell debris or dead cells, and only the supernatant was collected and filtered through a 0.2-µm filter. The filtrate containing only components having a size of 0.2 µm or less was collected, placed in a 100-kDa filter, and centrifuged at 10,000xg for 30 minutes to remove components having a molecular weight of 100 kDa or less, thus obtaining concentrated exosomes. PBS was added to the 100-kDa filter containing the concentrated exosomes and washed at 10000xg for 5 minutes, and this addition and washing process was repeated twice to remove the medium component from the exosome concentrate, thus obtaining exosomes.

### Experimental Example 1. Analysis of Telomere Elongation Effect Depending on Type of Cells

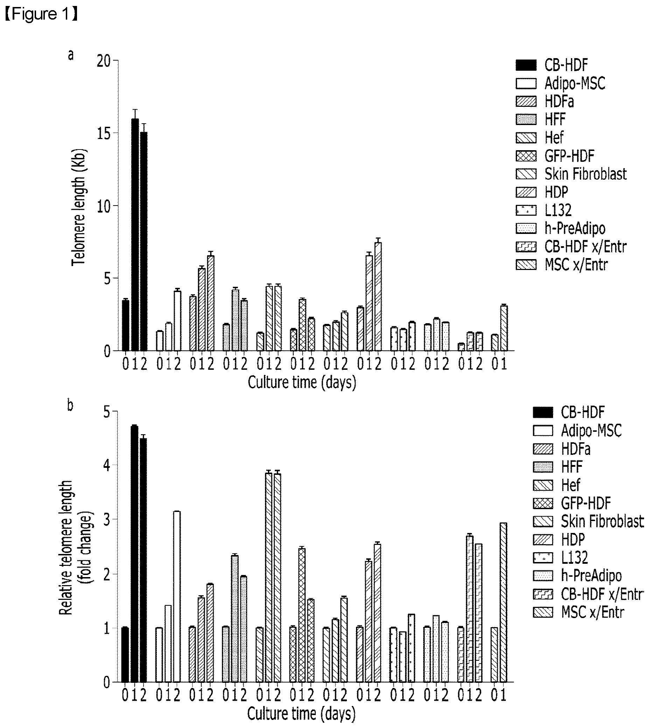

In order to compare the effect of Example 1 depending on the type of cells, ultrasound stimulation was added to each of the following types of 1×10⁶ cells in 1 ml of DMEM medium (fibroblast culture medium) according to the method of Example 1: CB-HDFs (CellBio), Adipo-MSC cells (professor Hwang Dong-Yeon Lab.), HDFa cells (Invitrogen), HFF cells (Cha University), Hef cells (Cha University), GFP-HDF cells (GFP-HNDFs, Angio-proteomie), skin fibroblasts (obtained from a 60-year-old stroke patient sample in compliance with the IRB-approved protocol), HDP cells (CellBio), L132 cells (ATCC), h-PreAdipo cells (ATCC), CB-HDF x/Entr cells, and MSC x/Entr cells (here, CB-HDF x/Entr and MSC x/Entr cells are pluripotent cells derived from CB-HDF and MSC cells, respectively, in human embryonic stem cell culture medium by application of a method of inducing pluripotent cells through ultrasound treatment in the study conducted by the present inventor (Lee et al., An ultra-effective method of generating extramultipotent cells from human fibroblasts by ultrasound, Biomaterials, 2017.)). Then, the cells were cultured for 0, 1 and 2 days, and the telomere lengths in the cells were analyzed according to a qPCR method using a kit (Absolute Human Telomere Length Quantification qPCR Assay Kit, Cat No. 8918, ScienCell^{™}). As a result, as shown in FIG. 1 and Table 1 below, it was confirmed that telomeres in all the types of cells were elongated.

**[Table 1]**

| Type of cell | Change (fold) in telomere length relative to control | |
|---|---|---|
| | Absolute value (kb) | Relative value (fold) |
| CB-HDF | 12.04 | 4.47 |
| MSC | 1.65 | 2.28 |
| HDFa | 2.36 | 1.79 |
| HFF | 2.00 | 2.14 |
| Hef | 3.22 | 3.80 |
| GFP-HDF | 2.36 | 1.79 |
| Skin Fibroblast | 0.57 | 1.34 |
| HDP | 4.05 | 2.40 |
| L132 | 1.30 | 1.54 |
| h-PreAdipo | 3.15 | 2.44 |
| CB-HDF x/Entr | 0.85 | 1.26 |
| MSC x/Entr | 1.82 | 2.93 |

### Experimental Example 2. Analysis of Telomere Elongation Effect Depending on Kind of Physical Stimulation

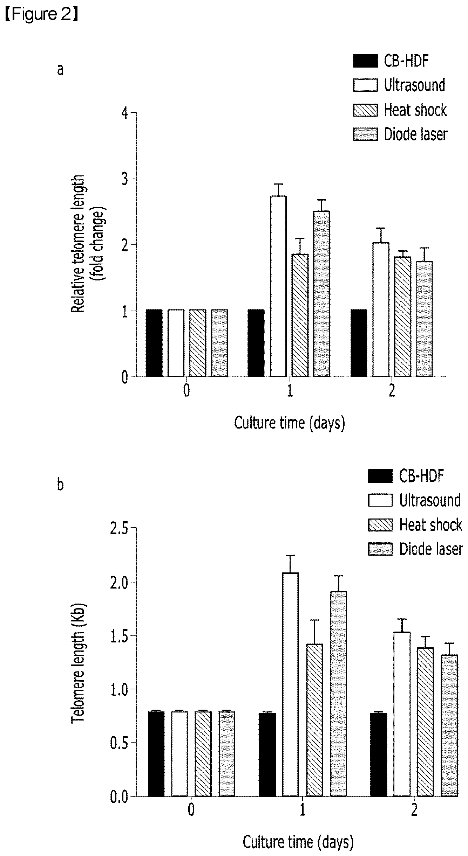

To analyze the effect of physical stimulation on telomere analysis depending on the kind of physical stimulation, each physical stimulation was added to CB-HDFs in human embryonic stem cell culture medium according to the methods of Examples 1 to 3, and then the cells were cultured for 0, 1 and 2 days, and the telomere length in the cells was analyzed according to a qPCR method. As a result, as shown in FIG. 2, it was confirmed that ultrasound, heat and light stimuli all elongated telomeres.

### Experimental Example 3. Analysis of Telomere Elongation Effect of indirect Ultrasound Stimulation

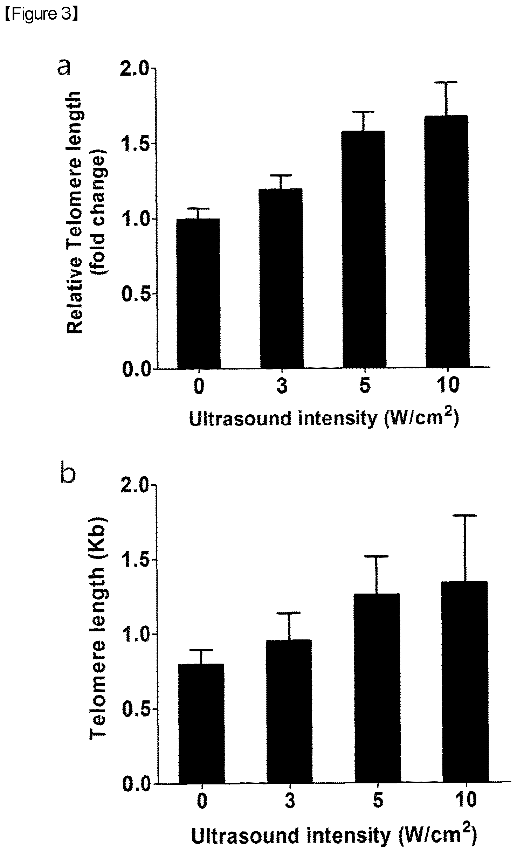

In order to examine whether telomeres are elongated even by indirect physical stimulation, not by direct physical stimulation, indirect ultrasound stimulation was applied to CB-HDFs according to the method of Example 2, and then the cells were cultured for 1 day, and the telomere length in the cells were analyzed according to a qPCR method. As a result, as shown in FIG. 3, it was confirmed that the effect on telomere elongation was great in proportion to the intensity of the ultrasound used.

### Experimental Example 4. Analysis of Telomere Elongation Effect After Ultrasound Treatment Depending on Medium Composition

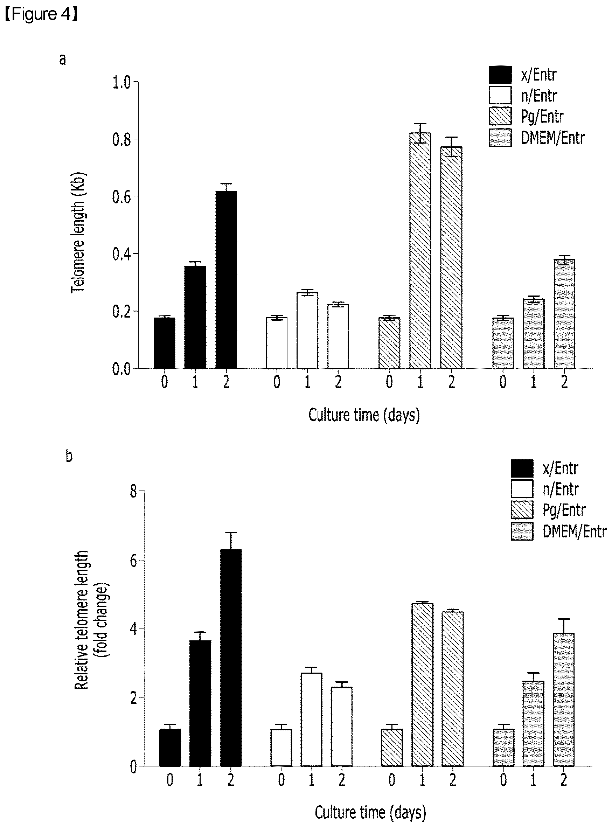

In order to compare the effect of Example 1 depending on the composition of medium, ultrasound was applied to 1×10⁶ CB-HDFs in 1 ml of each of human embryonic stem cell culture medium, neural stem cell culture medium, primary germ cell culture medium and DMEM medium according to the method of Example 1, and then the cells were cultured for 0, 1 and 2 days, and the telomere length in the cells was analyzed according to a qPCR method. As a result, as shown in FIG.4, it was confirmed that telomeres were elongated regardless of the composition of medium.

### Experimental Example 5. Analysis of Telomere Elongation Effect Depending on Number of Ultrasound Treatments

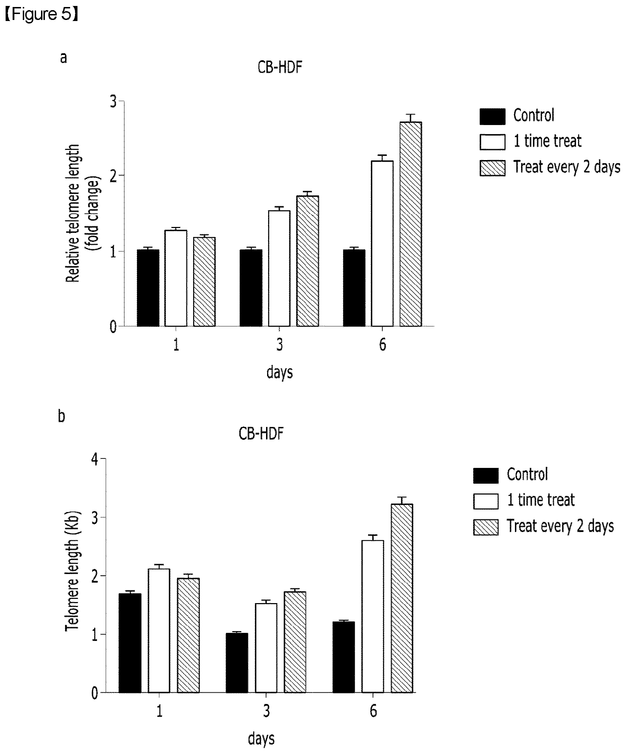

In order to examine whether a difference appears when the ultrasound according to Example 1 is repeated, ultrasound stimulation was applied to 1×10⁶ CB-HDFs in 1 ml of DMEM medium according to the method of Example 1, and the cells were cultured for 1, 3 and 6 days. In this case, the ultrasound stimulation was applied one time or applied every 2 days. The telomere length in the cells was analyzed according to a qPCR method. As a result, as shown in FIG. 5, it was confirmed that the telomere length increased regardless of the number of ultrasound treatments, and as the number of ultrasound treatments increased, the increment in the telomere length compared to the control not treated with ultrasound increased.

### Experimental Example 6. Analysis of TERT Gene Expression After Ultrasound Treatment Depending on Type of Cells

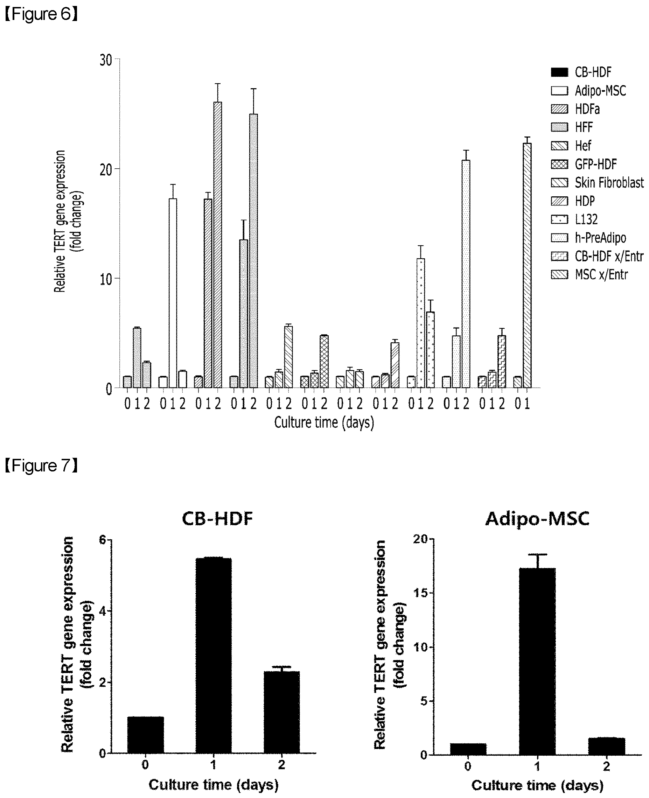

In order to examine whether the method of Example 1 shows a difference in the expression level of TERT gene depending on the type of cells, ultrasound stimulation was applied to each of the following types of 1×10⁶ cells according to the method of Example 1: CB-HDFs, Adipo-MSC cells, HDF cells, HFF cells, Hef cells, GFP-HDF cells, skin fibroblasts, HDP cells, L132 cells, h-PreAdipo cells, CB-HDF x/Entr cells and MSC x/Entr cells. Then, the cells were cultured for 0, 1 and 2 days, and changes in TERT gene expression in the cells were analyzed according to a qPCR method. As a result, as shown in FIG. 6 and Table 2 below, it was confirmed that the expression levels of TERT in all the types of cells increased. In addition, as shown in FIG. 7, it was shown that the expression levels of TERT in CB-HDFs and Adipo-MSC cells significantly increased on day 1 of culture after ultrasound treatment and then decreased again on day 2. That is, since continuous expression of TERT has a risk of development of cancer, etc., it is expected that temporary expression of TERT may ameliorate aging-related conditions caused by telomere shortening while reducing this risk.

**[Table 2]**

| Type of cell | Change (fold) in TERT gene expression relative to control |
|---|---|
| CB-HDF | 5.4 |
| MSC | 19.46 |
| HDFa | 21.61 |
| HFF | 16.81 |
| Hef | 3.50 |
| GFP-HDF | 3.05 |
| Skin Fibroblast | 1.53 |
| HDP | 2.66 |
| L132 | 6.88 |
| h-PreAdipo | 11.92 |
| CB-HDF x/Entr | 3.08 |
| MSC x/Entr | 22.20 |

### Experimental Example 7. Analysis of Change in Intracellular β-Catenin Gene Expression by Ultrasound Treatment

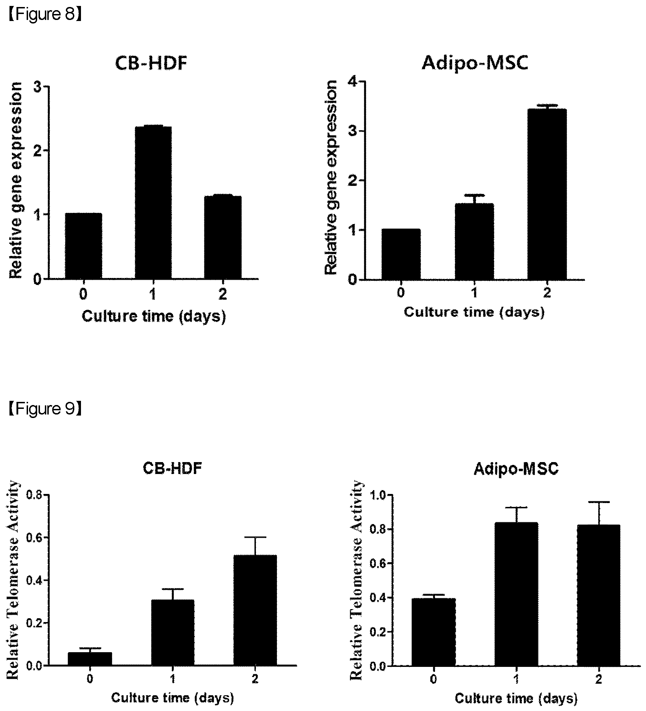

In order to examine how the telomere elongation method according to Example 1 affects the β-catenin gene which is a transcriptional activator of TERT, ultrasound stimulation was applied to 1×10⁶ CB-HDFs in 1 ml of DMEM medium and 1×10⁶Adipo-MSC cells in 1 ml of DMEM medium according to the method of Example 1, and then the cells were cultured for 0, 1 and 2 days, and changes in β-catenin gene expression in the cells were analyzed according to a qPCR method. As a result, as shown in FIG. 8, the expression levels of β-catenin increased in the two types of cells tested all increased.

### Experimental Example 8. Analysis of Change in Intracellular Telomerase Activity by Ultrasound Treatment

In order to examine how the telomere elongation method according to Example 1 affects telomerase activity, ultrasound stimulation was applied to 1×10⁶ CB-HDFs in 1 ml of DMEM medium and 1×10⁶Adipo-MSC cells in 1 ml of DMEM medium according to the method of Example 1, and then the cells were cultured for 0, 1 and 2 days, and telomerase activities in the cells were analyzed by Sciencell's Telomerase Activity Quantification qPCR kit (TAQ). As a result, as shown in FIG. 9, it was confirmed that telomerase activities in the two types of cells tested all increased.

### Experimental Example 9. FISH Analysis of Telomeres in Ultrasound-Treated Cells

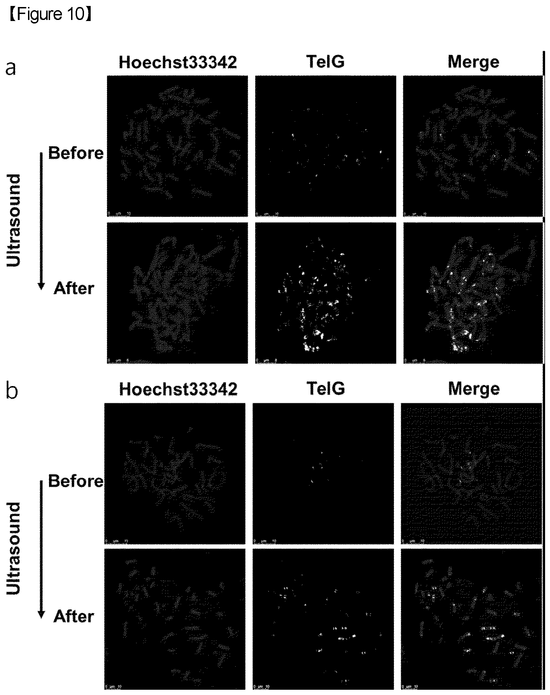

In order to further confirm that telomere elongation is induced by the telomere elongation method according to Example 1, ultrasound stimulation was applied to 1×10⁶ CB-HDFs in 1 ml of DMEM medium and 1×10⁶ Adipo-MSC cells in 1 ml of DMEM medium according to the method of Example 1, and then the cells were cultured for 1 day. Then, fluorescence *in situ* hybridization (FISH) analysis was performed using a TelG telomere probe (TTAGGGTTAGGGTTAGGG), and fluorescent signals were analyzed with a confocal microscope. At this time, the cells were counterstained with Hoechst 33342. As a result, as shown in FIG. 10, it was confirmed that the amounts of telomeres in the two types of cells tested all increased.

### Experimental Example 10. Cell Immunofluorescence Staining for Ki67 and TERT in Ultrasound-Treated Cells

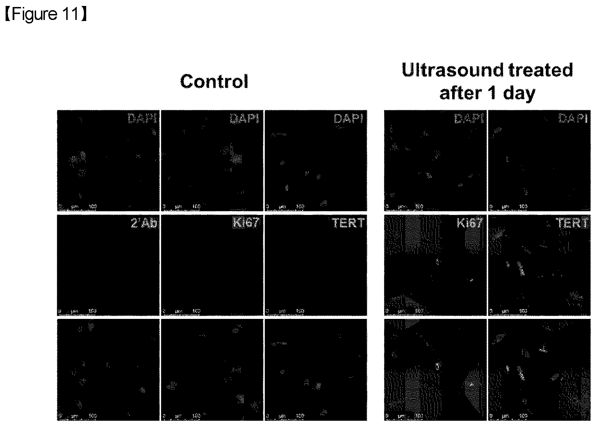

In order to confirm whether TERT gene expression induced by the telomere extension method according to Example 1 leads to an increase in protein expression, ultrasound stimulation was applied to 1×10⁶ CB-HDFs in 1 ml of DMEM medium according to the method of Example 1, and the cells were cultured for 1 day, and then fluorescence-stained using anti-Ki67 and anti-TERT antibodies and observed with a confocal microscope. As a result, as shown in FIG. 11, it was confirmed that, when the cells were treated with ultrasound, expression of the telomere-related TERT protein increased and expression of the cell division marker Ki67 protein also increased.

### Experimental Example 11. Analysis of Senescence-Related β-Galactosidase Activity

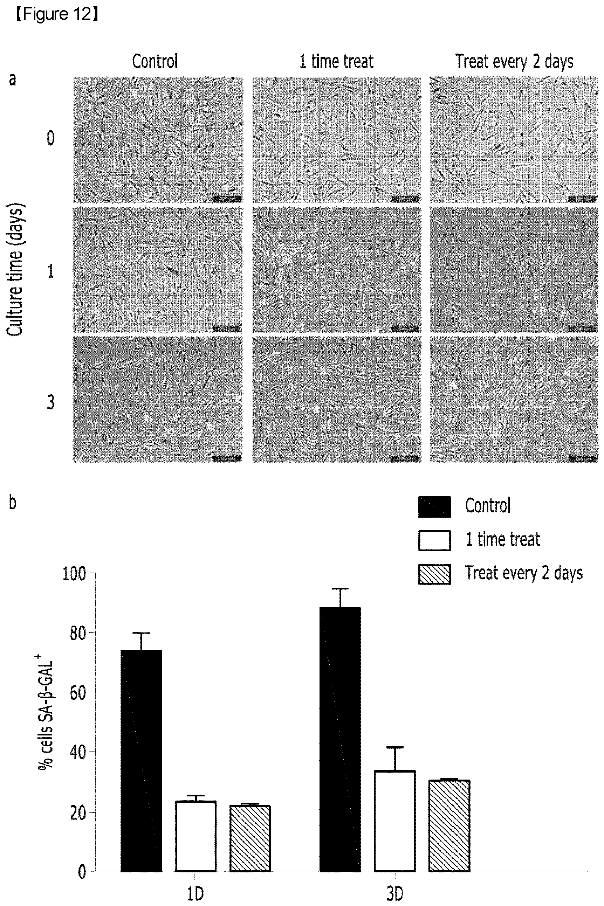

In order to examine how the above-described changes caused by the telomere elongation method according to Example 1 affects cell senescence, ultrasound stimulation was applied to 1×10⁶ CB-HDFs in 1 ml of DMEM medium according to the method of Example 1, and the cells were cultured for 1 day and 3 days. Then, a test for β-galactosidase activity known to have a correlation with intracellular senescence was performed using X-gal, followed by analysis with a phase contrast microscope. At this time, the ultrasound treatment was performed one time or performed every 2 days. As a result, as shown in FIG. 12, it could be confirmed that β-galactosidase activity decreased, suggesting that the method for elongating telomeres according to the present invention has an anti-senescence effect.

### Experimental Example 12. Analysis of Changes in Gene Expression in Cells through RNA-sea Analysis of Genes in Exosomes Secreted from Cells

In Korean Patent No. 10-1855967 which is the previous invention of the present inventors, changes in gene expression in cells and exosome secretion from cells could be induced by ultrasound treatment. Since secreted exosomes contain gene products expressed in cells, changes in gene expression in cells whose telomeres have been elongated by the method of Example 1 were analyzed through gene expression in exosomes secreted from the cells. As a result of RNA-seq, as shown in Table 3 below, it was confirmed that expression of telomerase activity-related genes and genes associated with telomere maintenance and protection in the exosomes secreted from the cells of the experimental group increased.

**[Table 3]**

| | Genes | Read count | |
|---|---|---|---|
| | | HDF.Exo | x.Exo |
| Telomerase activity-related genes | TNKS1BP1 | 0 | 17 |
| | PINX1 | 0 | 15 |
| | TERF1 | 0 | 7 |
| | RAD50 | 0 | 6 |
| | RFC1 | 0 | 5 |
| | TERF2IP | 0 | 5 |
| | DKC1 | 0 | 3 |
| | TERF2 | 0 | 3 |
| | TERT | 0 | 3 |
| Genes associated with telomere maintenance and protection | TEP1 | 0 | 94 |
| | PRKDC | 0 | 33 |
| | ERCC4 | 0 | 23 |
| | XRCC5 | 0 | 19 |
| | XRCC6 | 0 | 18 |
| | TNKS1BP1 | 0 | 17 |
| | PINX1 | 0 | 15 |
| | BLM | 0 | 11 |
| | SMG6 | 0 | 11 |
| | PTGES3 | 0 | 10 |
| | PARP1 | 0 | 7 |
| | TERF1 | 0 | 7 |
| | RAD50 | 0 | 6 |
| | RFC1 | 0 | 5 |
| | TERF2IP | 0 | 5 |
| | HSPA1L | 0 | 4 |
| | NBN | 0 | 4 |
| | ACD | 0 | 3 |
| | DKC1 | 0 | 3 |
| | TERF2 | 0 | 3 |
| | TERT | 0 | 3 |

The above description of the present invention is exemplary, and those of ordinary skill in the art will appreciate that it can be easily modified into other specific forms.

## Claims

1. A method for elongating telomeres of cells, the method comprising steps of:
providing physical stimulation directly or indirectly to the cells; and
culturing a mixture of the cells and a medium for a predetermined time,
wherein the providing the stimulation directly to the cells is applying the physical stimulation to a medium containing the cells, and the providing the stimulation indirectly to the cells is applying the physical stimulation to the medium not containing cells and then mixing the medium and the cells,
wherein the physical stimulation is any one selected from among direct or indirect ultrasound stimulation,
wherein the providing the ultrasound stimulation directly to the cells is performed at an ultrasound intensity of 0.1 to 3 W/cm² and a frequency of 20 kHz to 20 MHz for a duration of 0.1 seconds to 20 minutes, and the providing the ultrasound stimulation indirectly to the cells is performed at an ultrasound intensity of 1 to 20 W/cm² and a frequency of 20 kHz to 20 MHz for a duration of 0.1 seconds to 20 minutes.

2. The method of claim 1, wherein the cells are selected from the group consisting of mammalian stem cells, progenitor cells, fibroblasts, keratinocytes or organ tissue cells.

3. The method of claim 1, wherein the medium is selected from among a culture medium or a differentiation-inducing medium.

4. The method of claim 1, wherein the culturing of the mixture is performed for 1 hour to 10 days.

5. The method of claim 1, wherein expression of one or more of TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 and β-catenin genes in the cells after the culturing increases compared to that before the culturing.

6. The method of claim 1, wherein telomerase activity in the cells after the culturing increases compared to that before the culturing.

7. The method of claim 1, wherein β-galactosidase activity in the cells after the culturing decreases compared to that before the culturing.

## Patentansprüche

1. Verfahren zur Verlängerung von Telomeren von Zellen, wobei das Verfahren die folgenden Schritte umfasst:
direktes oder indirektes Bereitstellen physikalischer Stimulierung an die Zellen; und
Kultivieren eines Gemischs der Zellen und eines Mediums über eine vorbestimmte Zeit,
wobei beim direkten Bereitstellen der Stimulierung an die Zellen die physikalische Stimulierung an ein die Zellen enthaltendes Medium angelegt wird und beim indirekten Bereitstellen der Stimulierung an die Zellen die physikalische Stimulierung an das die Zellen nicht enthaltende Medium angelegt wird und danach das Medium und die Zellen gemischt werden,
wobei es sich um eine unter direkter bzw. indirekter Ultraschallstimulierung ausgewählte physikalische Stimulierung handelt,
wobei das direkte Bereitstellen der Ultraschallstimulierung an die Zellen bei einer Ultraschallintensität von 0,1 bis 3 W/cm² und einer Frequenz von 20 kHz bis 20 MHz über eine Zeitdauer von 0,1 Sekunden bis 20 Minuten erfolgt und das indirekte Bereitstellen der Ultraschallstimulierung an die Zellen bei einer Ultraschallintensität von 1 bis 20 W/cm² und einer Frequenz von 20 kHz bis 20 MHz über eine Zeitdauer von 0,1 Sekunden bis 20 Minuten erfolgt.

2. Verfahren nach Anspruch 1, wobei die Zellen aus der Gruppe bestehend aus Säuger-Stammzellen, -Vorläuferzellen, -Fibroblasten, -Keratinozyten oder -Organgewebezellen ausgewählt sind.

3. Verfahren nach Anspruch 1, wobei das Medium unter einem Kulturmedium bzw. einem Differenzierung induzierendem Medium ausgewählt ist.

4. Verfahren nach Anspruch 1, wobei das Gemisch 1 Stunde bis 10 Tage lang kultiviert wird.

5. Verfahren nach Anspruch 1, wobei die Expression eines oder mehrerer der Gene TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 und S-Catenin in den Zellen nach dem Kultivieren gegenüber der vor dem Kultivieren zunimmt.

6. Verfahren nach Anspruch 1, wobei Telomerase-Aktivität in den Zellen nach dem Kultivieren gegenüber der vor dem Kultivieren zunimmt.

7. Verfahren nach Anspruch 1, wobei β-Galactosidase-Aktivität in den Zellen nach dem Kultivieren gegenüber der vor dem Kultivieren abnimmt.

## Revendications

1. Procédé d'allongement de télomères de cellules, le procédé comprenant les étapes suivantes :
la fourniture d'une stimulation physique directement ou indirectement aux cellules ; et
la culture d'un mélange des cellules et d'un milieu pendant une durée prédéterminée,
dans lequel la fourniture de la stimulation directement aux cellules consiste à appliquer la stimulation physique à un milieu contenant les cellules, et la fourniture de la stimulation indirectement aux cellules consiste à appliquer la stimulation physique au milieu ne contenant pas de cellules, puis à mélanger le milieu et les cellules, dans lequel la stimulation physique est l'une quelconque sélectionnée parmi une stimulation ultrasonore directe ou indirecte,
dans lequel la fourniture de la stimulation ultrasonore directement aux cellules est effectuée à une intensité ultrasonore de 0,1 à 3 W/cm² et une fréquence de 20 kHz à 20 MHz pendant une durée de 0,1 seconde à 20 minutes, et la fourniture de la stimulation ultrasonore indirectement aux cellules est effectuée à une intensité ultrasonore de 1 à 20 W/cm² et une fréquence de 20 kHz à 20 MHz pendant une durée de 0,1 seconde à 20 minutes.

2. Procédé selon la revendication 1, dans lequel les cellules sont sélectionnées dans le groupe consistant en les cellules souches de mammifère, les cellules progénitrices, les fibroblastes, les kératinocytes ou les cellules de tissus organiques.

3. Procédé selon la revendication 1, dans lequel le milieu est sélectionné parmi un milieu de culture ou un milieu induisant une différenciation.

4. Procédé selon la revendication 1, dans lequel la culture du mélange est effectuée pendant 1 heure à 10 jours.

5. Procédé selon la revendication 1, dans lequel l'expression d'un ou plusieurs des gènes TERT, TERF2, DKC1, TERF2IP, RFC1, RAD50, TERF1, PINX1, TNKS1BP1, ACD, NBN, HSPA1L, PARP1, PTGES3, SMG6, BLM, XRCC5, XRCC6, ERCC4, PRKDC, TEP1 et de la β-caténine dans les cellules après la culture augmentent par rapport à celle avant la culture.

6. Procédé selon la revendication 1, dans lequel l'activité de la télomérase dans les cellules après la culture augmente par rapport à celle avant la culture.

7. Procédé selon la revendication 1, dans lequel l'activité de la β-galactosidase dans les cellules après la culture diminue par rapport à celle avant la culture.
